# EUROPEAN PATENT SPECIFICATION

(11) **EP 1 830 848 B1**
(45) Date of publication and mention of the grant of the patent: **16.12.2009**
(21) Application number: 05820657.4
(22) Date of filing: 19.12.2005
(51) Int. Cl.: A61K 31/427, A61K 31/5377, C07D 417/06, C07D 417/14, A61P 35/00, A61P 19/02, A61P 17/06, A61P 17/00, A61P 27/02, A61P 27/06

(54) **USE OF THIAZOLIDINONE DERIVATIVES FOR THE TREATMENT OF SOLID TUMORS**
VERWENDUNG VON THIAZOLIDINON-DERIVATEN ZUR BEHANDLUNG VON FESTEN TUMOREN
UTILISATION DE DERIVES DE THIAZOLIDINONE POUR LE TRAITEMENT DE TUMEURS SOLIDES

(30) Priority: 23.12.2004 IT MI20042475
(43) Date of publication of application: 12.09.2007
(73) Proprietor: Cell Therapeutics Europe S.R.L., 20091 Bresso (IT)
(72) Inventor: CASSIN, Mara, I-20091 Bresso (IT); COLELLA, Gennaro, I-20091 Bresso (IT); DE MUNARI, Sergio, I-20091 Bresso (IT); GRUGNI, Mario, I-20091 Bresso (IT)
(74) Representative: Banfi, Paolo
(86) International application number: PCT/EP2005/013650
(87) International publication number: WO 2006/066846

(56) References cited:
- WO-A-98/53790
- WO-A-03/105840
- WO-A-2004/024058
- WO-A-2004/028535
- WO-A-2004/043955
- WO-A-2005/007141
- WO-A-2005/016227
- WO-A-2005/076695
- FR-A- 2 169 334
- US-A1- 2004 002 526
- US-A1- 2004 266 846
- DATABASE WPI 2 November 1999 (1999-11-02), Derwent Publications Ltd., London, GB; Class 000,page 7, AN 2000-075331 XP002378133 KOBAYASHI K., NISHIYAMA T., NAKADE S.: "Thiazolidine derivative, and medicine containing the same as active ingredient" & JP 11 302280 A (ONO PHARMACEUTICAL CO) 2 November 1999 (1999-11-02) -& Computer-generated translation of JP11302280 XP002378169
- NICOLAUS B J R: "Symbiotic Approach to Drug Design" DECISION MAKING IN DRUG RESEARCH, 1983, pages 173-186, XP002197412
- DATABASE WPI 24 March 2005 (2005-03-24), Derwent Publications Ltd., London, GB; Class 052,page 5, AN 2005-242351 XP002378134 ITAI A, KUBO A, MUTO S, SOTOME T, YAMAGUCHI Y: "plasminogen activator inhibitor-1 inhibitor" & WO 2005/026127 A (INST MEDICINAL MOLECULAR DESIGN INC) 24 March 2005 (2005-03-24) -& WO 2005/026127 A (INSTITUTE OF MEDICINAL MOLECULAR DESIGN. INC; MUTO, SUSUMU; KUBO, ASAK) 24 March 2005 (2005-03-24)

## Description

### Field of the invention

The invention relates to thiazolidinone compounds capable of inhibiting the interaction between HIF-1α transcription factor and its coactivator p300 and of preventing the production of Vascular Endothelial cell Growth Factor.

### Background of the invention

Vascular Endothelial Cell Growth Factor plays a key role in the processes of physiological and physiopathological angiogenesis. A number of mechanisms are involved in the regulation of the VEGF gene, among which a fundamental role is played by the tissue oxygen tension, as proved by the reversible increase in VEGF mRNA levels under *in vivo* and *in vitro* hypoxia conditions. Increased expression of VEGF mRNA is mainly mediated by the transcription factor HIF-1 (hypoxia-inducible factor-1), which binds to a recognition site in the promoter region of the VEGF gene.

A great number of experimental data show that HIF-1 is a global regulator of oxygen homeostasis and that an impaired activity of HIF-1 promotes survival, proliferation, invasion and metastatization of tumoral cells (1). It has been therefore suggested that therapeutic strategies focusing on the inhibition of HIF-1 activity could increase the survival of cancer patients (2).

HIF-1 is a heterodimer consisting of HIF-1α and HIF-1β sub-units, which dimerize and bind to DNA through the bHLH-PAS domain (3). The expression of the HIF-1α sub-unit is strictly regulated by the tissue oxygen tension (4) through processes of ubiquitination and proteasome degradation, mediated by the binding of VHL protein to HIF-1α. Such interaction only takes place when HIF-1α has been hydroxylated at the 402 and 564 proline residues. Oxygen is the limiting substrate for prolyl-hydroxylase which modifies HIF-1α (5). The expression of HIF-1α exponentially increases as O₂, concentration decreases and determines the HIF-1 global activity levels.

The function of HIF-1α transactivation domain is also subject to negative regulation, controlled by oxygen partial pressure. The N-terminal transactivation domain is negatively regulated through the recruitment of hystone deacylase by VHL and by HIF-1 inhibiting factor (FIH-1), which binds to both VHL and HIF-1α (6).

HIF-1 activation takes place through p300/CBP coactivators which physically interact with the activation of the HIF-1 domain to promote transcription of genes like VEGF (7). Both p300 and CBP are co-activators also for other transcription factors, such as Stat-3, NF-κB, p53.

The interaction of p300/CBP with HIF-1 is essential to transcription, and recent publications have proved the importance of the HIF-1/p300 interaction for tumor growth (8). HIF-1α C-terminal trans-activation domain (C-TAD) binds to a p300 and CBP domain known as CH1. The binding of CBP and p300 to HIF-1α is negatively regulated through oxygen-dependent hydroxylation of asparagine 803 in the C-terminal activation domain by FIH-1. Thus, hypoxia causes both stabilization to proteasome degradation and transcriptional activity of HIF-1.

Structural details of the interaction between HIF-1α TAD-C and the CH1 domain of p300 or CBP have been elucidated (9, 10). Details of the interaction between p300/CBP and the CITED2 protein (also known as p35^{srj}), which is considered a negative regulator of Hif-1α activity (11), have also been published.

HIF-1 activation induces the transcription of a number of genes involved in the production of angiogenic factors, glucose carriers, glycolytic enzymes, survival, migration and invasion factors, which are particularly important for tumor progression.

Aberrant expression of Hif-1α was observed in more than 70% of human tumors and their metastases and was connected with an increase in vascularization and tumor progression (12-14). In clinical practice, aberrant expression of Hif-1α was associated to therapy failure and mortality increase in a number of tumoral pathologies, such as non-small cells lung carcinoma (15), oropharyngeal squamous cell cancer (16), early-stage cervical cancer (17), head-and-neck cancer (18), mutated p53 ovary cancer (19), oligodendrioglioma (20) and BCL-2 positive esophageal cancer (21).

Various approaches for inhibiting HIF-1 activity have been described in the literature. Some of them suggested the use of antisense oligonucleotides for Hif-1α or negative dominant forms of Hif-1α.

Among the pharmacological approaches, Hif-1α activity inhibitors acting through indirect mechanisms have been described, such as PI3K-mTOR inhibitors (22-23) and MEKK (24) inhibitors which act on the transduction of signals controlling Hif-1α activity; inhibitors of HSP90 chaperone protein (25); thioredoxin reductase inhibitors, which act modifying the cell redox state (26); molecules which destabilize microtubules, such as 2-methoxyestradiol (27) and epothilones (28). Recently, both constitutive and hypoxia-induced inhibition of Hif-1α levels by PX-478 (Melphalan N-oxide) in human tumors transplanted in nude mice was reported. The compound shows marked antitumoral effects. However, the mechanism of action of this compound has yet to be completely clarified (29).

Finally, chaetomine, a dithiodioxopiperazine metabolite of Chaetomium sp fungi, has recently been reported to interfere with the binding of Hif-1α to p300. The compound acts altering the CH1 domain structure of p300, thus preventing its interaction with Hif-1α. Chaetomine administration to tumor-bearing mice inhibits hypoxia-induced transcription in the tumor and tumor growth (30). It would therefore be advantageous to provide further compounds capable of inhibiting the binding between HIF-1α to p300.

US 2004/002526 A1 discloses thiazolidinone compounds for use as inhibitors of phospholipase D and WO 98/53790 WO 2004/028535 and WO 2004/043955 disclose thiazolidinones useful as antitumor agents. JP 11302280 discloses thiaxolidinones as sialyl Lewis X synthesis inhibitors for the treatment of, inter alia, psoriasis and cancer.

### Disclosure of the invention

It has now been found that some thiazolidinones inhibit the interaction between Hif-1α and p300 and prevent VEGF production in tumour cells under hypoxia conditions. The compounds are therefore useful for the control of angiogenesis and tumor growth.

Accordingly, the present invention relates to the use of a compound selected from:
2-[5-(3-benzyl₋4-oxo-2-thioxo-thiazolidine-5-ylidenemethyl)-furan-2-yl]benzoic acid methyl ester (1);
4-[5-(3-benzyl-4-oxo-2-thioxo-thiazolidine-5-ylidenemethyl)furan-2-yl-] benzonitrile (**5**);
4-{ 5-[3-(4-methylbenzyl)-4-oxo-2-thioxo-thiazolidine-5-ylidenemethyl]furan-2-yl-}benzonitrile (**6**);
4-{5-[3-(4-methylbenzyl)-4-oxo-2 thioxo-thiaxolidine-5-ylidenemethyl]-furan-2-yl}benxoic acid methyl ester (**9**);
4-{ 5-[3-(3,4-methylenedioxybenzyl)-4-oxo-2-thioxo-thiazolidine-5-ylidenemethyl]-furan-2-yl}benzenesulfonamide (**11**);
4-{5-[3-(3,4-methylenedioxybenzyl)-4-oxo-2-thioxo-thiazolidine-5-ylidenemethyl]-furan-2-yl}benzoic acid methyl ester (**12**);
4-{5-[3-(2-thienyl)methyl-4-oxo-2-thioxo-thiazolidine-5-ylidenemethyl]-furan-2-yl}benzonitrile (**14**);
4-{5-[3-(2-thienyl)methyl-4-oxo-2-thioxo-thiazolidine-5-ylidenemethyl]-furan-2-yl}benzenesulfonamide (**15**);
4-{5-[3-(3,4-dimethoxyphenyl)-4-oxo-2-thioxo-thiazolidine-5-ylidenemethyl]-furan-2-yl}benzoic acid (**17**);
4-{5-[3-[2-(3,4-dimethoxyphenyl)ethyl]-4-oxo-2-thioxo-thiazolidine-5-ylidenemethyl]-furan-2-yl}benzonitrile (**18**);
4-{5-[3-[2-(phenyl)ethyl]4-oxo-2-thioxo-thiazolidine-5-ylidenemethyl]-furan-2-yl}benzonitrile (**19**);
4-{ 5-[3-[2-(phenyl)ethyl]-4-oxo-2-thioxo-thiazolidine-5-ylidenemethyl]-furan-2-yl}benzoic acid methyl ester (**20**);
4-{5-[3-[2-(3,4-dimethoxyphenyl)ethyl]-4-oxo-2-thioxo-thiazolidine-5-ylidenemethyl]-furan-2-yl}benzoic acid methyl ester (**21**);
2-{5-[4-[3-2-(phenyl)ethyl]-4-oxo-2-thioxo-thiazolidine-5-ylidenemethyl]-furan-2-yl]phenoxy}ethanol (**22**);
3-benzyl-5-[[5-(2-methoxyphenyl)furan-2-ylidenemethyl]-4-oxo-2-thioxo-thiazolidine (**23**);
2-[5-(3-benzyl-4-oxo-2-thioxo-thiazolidine-5-ylidenemethyl)-furan-2-yl]benzoic acid (**24**), for the preparation of pharmaceutical compositions for the treatment of solids tumors.

The above-mentioned compounds, except for compound **1**, are a further object of the invention.

The compounds of the invention can be synthesised according to known methods, for example those described in US 2004/0002526 A1, which comprise the condensation between a 2-thioxo-thiazolidine-4-one, suitably substituted at the 3- position and a suitably substituted furanaldehyde, or according to the methods described in Tetrahedron Lett., 44, 4257 (2003), Bioorg. Med. Chem. Lett., 12, 2681 (2002), J. Med. Chem., 41, 2390 (1998).

The following schemes illustrate in particular the synthesis 2-[5-(3-benzyl)-4-oxo-2-thioxo-thiazolidine-5-ylidenemethyl]-furan-2-yl}benzoic acid methyl ester.

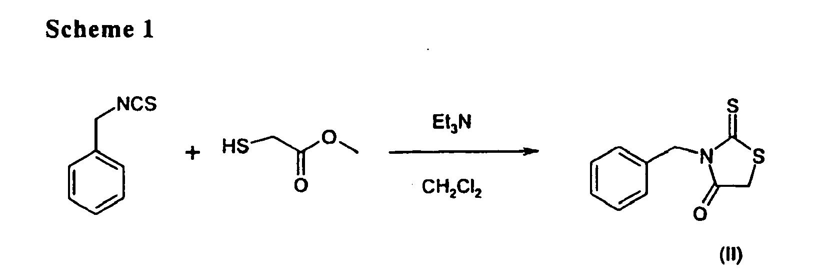

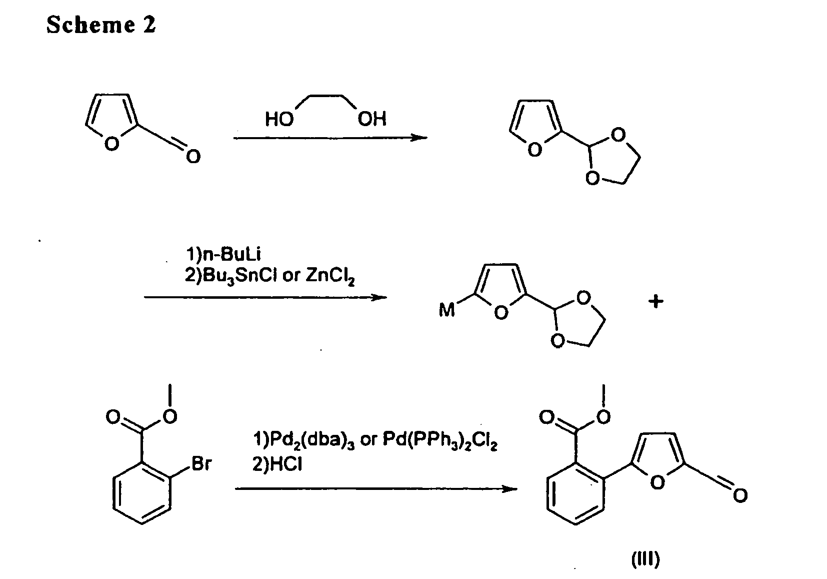

Wherein M is Li, Zn or Bu₃Sn and DBA is dibenzylidenacetone Alternatively, compound (III) can be synthesized according to the following schemes:

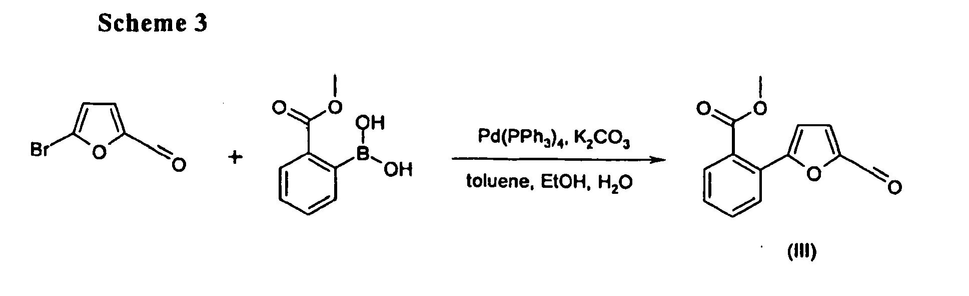

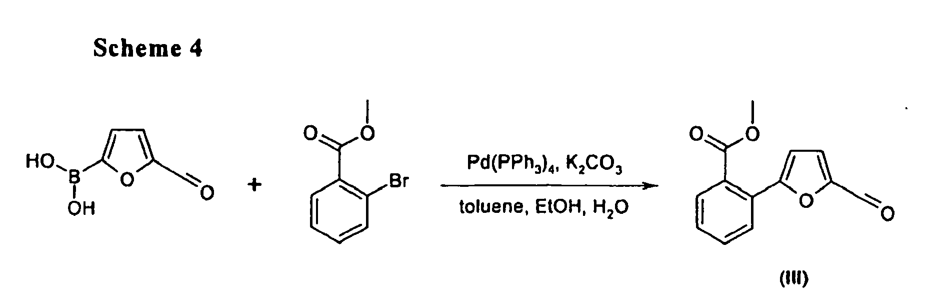

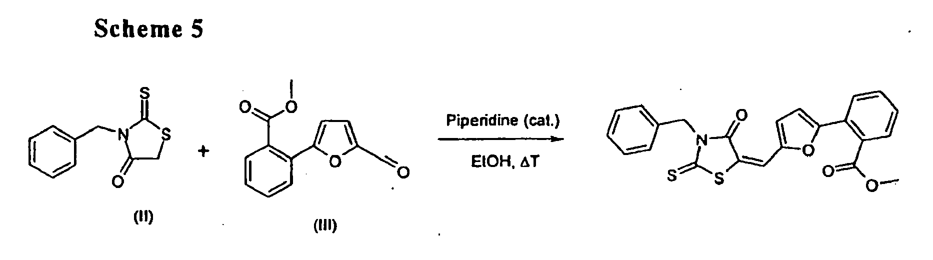

All the reagents reported in Schemes 1-5 can be prepared with known methods and are commercially available.

The compounds of the invention inhibit the interaction between HIF-1α and p300 with IC₅₀ ranging from 0.5 to 25 µM and the production of VEGF in tumour cells under hypoxia conditions with IC₅₀ ranging from 0.10 to 100 µM.

The compounds can therefore be used for the preparation of pharmaceutical compositions for the treatment of solid tumors. The compositions can be solid, semi-solid or liquid, preferably in the form of solutions, suspensions, powders, granules, tablets, capsules, syrups, suppositories, aerosol or controlled-release systems. The compositions can be administered through different routes, in particular through the oral, transdermal, subcutaneous, intravenous, intramuscular, rectal and intranasal route. The amount of active ingredient per dosage unit depends on the form and on the administration route, the compound, the disease to treat, but in general ranges from 0.1 to 1000 mg, preferably from 1 to 600 mg. The principles and methods for the preparation of pharmaceutical compositions are known to those skilled in the art and are described, for example, in Remington's Pharmaceutical Science, Mack Publishing Company, Easton (PA).

The compounds of the invention, suitably formulated, can be used for the treatment of a number of pathologies in which angiogenesis is involved as pathogenesis factor, for example different forms of solid tumors, diabetic retinopathy, rheumatoid arthritis, psoriasis, emangioma, sclerodermia, neovascular glaucoma. Solid tumors particularly sensitive to the compounds of formula (**I**) comprise lung carcinoma, mammary carcinoma, prostate carcinoma, neuroblastoma, glioblastoma multiforme, melanoma, central nervous system cancer, squamous cell oropharyngeal cancer, cervical cancer, ovary, esophageal, kidney, colon, head-and-neck cancer and oligodendrioglioma.

The invention will be illustrated in greater detail in the following experimental section.

### EXPERIMENTAL SECTION

### Synthesis of the compounds

### General procedure A

### Example - 4-(5-Formyl-furan-2-yl)-benzoic acid methyl ester

A solution of 5-bromofuraldehyde (2.43 g, 13.9 mmoles), 4-(methoxycarbonyl)phenylboronic acid (2.50 g, 13.9 mmoles), tris(dibenzylideneacetone)palladium (0) (192 mg, 0.2 mmoles) and potassium fluoride (2.42 g, 41.7 mmoles) in 1,4-dioxane (100 ml) was added with a solution of tri-tert-butylphosphine in hexane (10% by weight, 101 mg, 0.5 mmoles). After heating at 65-70°C for 4 hours, the mixture was cooled to room temperature and treated with methylene chloride (150 ml). After stirring for 10 minutes, the mixture was filtered through Celite and the filtrate was concentrated under reduced pressure. The residue was purified by flash silica gel chromatography eluting with an ethyl acetate-hexane mixture (1:1) to give 4-(5-formyl-furan-2-yl)-benzoic acid methyl ester (2.6 g, 81 % yield).
¹H NMR (300 MHz, CDCl₃): δ 9.70 (s, 1H), 8.10 (d, 2H), 7.90 (d, 2H), 7.35 (d, 1H), 6.95 (d, 1H), 3.98 (s, 3H).

### General procedure B

### Example - 3-(3-Morpholino-4-yl-propyl)-2-thioxo-thiazolidine-4-one (reference)

A suspension of bis(carboxymethyl)trithiocarbonate (498 mg, 2.2 mmoles), potassium carbonate (138 mg, 1.0 mmoles) and 4-(3-aminopropyl)morpholine (288 mg, 2.0 mmoles) in water (20 ml) was refluxed for 12 hours. After addition of water (10 ml), the mixture was cooled to room temperature and extracted with 10% methanol-methylene chloride (3x50 ml). The combined extracts were dried over anhydrous magnesium sulphate and concentrated under reduced pressure. The residue was purified by flash silica gel chromatography eluting with 10% methanol-methylene chloride to give 3-(3-morpholino-4-yl-propyl)-2-thioxo-thiazolidine-4-one (252 mg, 48% yield). ¹H NMR (300 MHz, CDCl₃): δ 4.10 (dd, 2H), 3.95 (s, 2H), 3.55-3.70 (m, 4H), 2.30-2.50 (m, 6H), 1.70-1.90 (m, 2H).

### General procedure C

### Example - 4{5-[3-(3-Morpholino-4-yl-propyl)-4-oxo-2-thioxothiazolidine-5-ylidenemethyl]-furan-2-yl]-benzoic acid methyl ester (reference)

A solution of 4-(5-formyl-furan-2-yl)-benzoic acid methyl ester (59 mg, 0.27 mmoles) and 3-(3-morpholino-4-yl-propyl)-2-thioxo-thiazolidine-4-one (72 mg, 0.27 mmoles) in ethanol (10 ml) was added with piperidine (1 drop). After heating under reflux for 6 hours, the mixture was concentrated under reduced pressure. The residue was purified by flash silica gel chromatography eluting with 10% methanol-methylene chloride to give the title compound (32 mg, 25%yield).
¹H NMR (300 MHz, DMSO-d₆): δ 8.15 (d, 2H), 7.75 (d, 2H), 7.50 (s, 1H), 6.95-7.05 (m, 2H), 4.20 (t, 2H), 3.95 (s, 3H), 3.90 (t, 2H), 3.58-3.75 (m, 2H), 2.30-2.60 (m, 6H), 1.80-2.00 (m, 2H).

### 3-Benzyl-2-thioxo-thiazolidine-4-one

Following general procedure B, bis(carboxymethyl)-trithiocarbonate (2.50 g, 11.0 mmoles), was reacted with benzylamine (1.07 g, 10.0 mmoles). The title compound was obtained after flash silica gel chromatography eluting with 70% methylene chloride-hexane, (960 mg, 43% yield).

### 4-(5-Formyl-furan-2-yl)-benzonitrile

Following general procedure A, 5-bromo furaldehyde (3.50 g, 0.2 moles) was reacted with 4-cyanoboronic acid (2.94 g, 0.2 moles). After usual work-up of the mixture and chromatographic purification on silica gel column eluting with 50% ethyl acetate-hexane the title compound was obtained (2.60 g, 66% yield).
¹H NMR (300 MHz, CDCl₃): δ 9.70 (s, 1H), 8.00 (d, 2H), 7.80 (d, 2H), 7.40 (d, 1H), 7.00 (d, 1H).

### 2-Thioxo-3-(3,4-dimethoxy-phenylethyl)-thiazolidine-4-one

Following general procedure B, bis(carboxymethyl) thiocarbonate (905 mg, 4.0 mmoles), was reacted with 3,4-dimethoxy-benzylamine (363 mg, 2.0 mmoles) and, after flash silica gel chromatography eluting with 70% methylene chloride-hexane, the title compound was obtained (420 mg, 71% yield).
¹H NMR (300 MHz, CDCl₃): δ 6.70-6.90 (m, 3H), 4.20 (t, 2H), 4.00 (s, 2H), 3.90 (s, 3H), 3.85 (s, 3H), 2.95 (t, 2H).

### 4-(5-{3-[2-(3,4-Dimethoxy-phenyl)-ethyl]-oxo-2-thioxo-thiazolidine-5-ylidenemethyl}-furan-2-yl)-benzonitrile

Following general procedure C, 4-(5-formyl-furan-2-yl)-benzonitrile (39.4 mg, 0.2 mmoles) was reacted with 2-thioxo-3-(3,4-dimethoxy-benzyl)-thiazolidine-4-one (59.4 mg, 0.2 mmoles) to give the title compound (58 mg, 61 % yield).
¹H NMR (300 MHz, DMSO-d₆): δ 7.95-8.25 (m, 4H), 7.75 (s, 1H), 7.60 (d, 1H), 7.40 (d, 1H), 6.7-6.90 (m, 3H), 4.25 (t, 2H), 3.75 (s, 3H), 3.70 (s, 3H), 2.95 (s, 2H).

### 3-(2-Phenylethyl)-2-thioxo-thiazolidine-4-one

Following general procedure B, bis(carboxymethyl)trithiocarbonate (905 mg, 4.0 mmoles), was reacted with 2-phenylethylamine (242.4 mg, 2.0 mmoles). The title compound was obtained after flash silica gel chromatography eluting with 70% methylene chloride-hexane, (246 mg, 52% yield).
¹HMR (300 MHz, CDCl₃): δ 7.15-7.40 (m, 5H), 4.20 (t, 2H), 3.95 (s, 2H), 3.00 (t, 2H).

### 4-[5-(4-Oxo-3-(2-phenylethyl)-2-thioxo-thiazolidine-5-ylidenemethyl)-furan-2-yl]-benzonitrile

Following general procedure C, 4-(5-formyl-furan-2-yl)-benzonitrile (39.4 mg, 0.2 mmoles) was reacted with 3-(2-phenylethyl)-2-thioxo-thiazolidine-4-one (47.4 mg, 0.2 mmoles) to give the title compound (64 mg, 76% yield).
¹H NMR (300 MHz, DMSO-d₆): δ 7.95-8.25 (m, 4H), 7.75 (s, 1H), 7.55 (d, 1H), 7.40 (d, 1H), 7.10-7.35 (m, 5H), 4.24 (t, 2H), 3.00 (t, 2H).

### 4-[5-(4-Oxo-3-(2-phenylethyl)-2-thioxo-thiazolidine-5-ylidenemethyl)-furan-2-yl]-benzoic acid methyl ester

Following general procedure C, 4-(5-formyl-furan-2-yl)-benzoic acid methyl ester (46.0 mg, 0.2 mmoles) was reacted with 3-(2-phenylethyl)-2-thioxo-thiazolidine-4-one (47.4 mg, 0.2 mmoles) to give the title compound (39.2 mg, 44% yield).
¹H NMR (300 MHz, CDCl₃): δ 8.00 (d, 2H), 7.65 (d, 2H), 6.70-7.15 (m, 8H), 4.15 (t, 2H), 3.88 (s, 3H), 2.85 (t, 2H).

### 4-(5-{3-[2-(3,4-Dimethoxyphenyl)-ethyl]-4-oxo-2-thioxo-thiazolidine-5-ylidenemethyl}-furan-2-yl)-benzoic acid methyl ester

Following general procedure C, 4-(5-formyl-furan-2-yl)-benzoic acid methyl ester (46.0 mg, 0.2 mmoles) was reacted with 2-thioxo-3-[2-(3,4-dimethoxy-phenylethyl)]-thiazolidine-4-one (59.4 mg, 0.2 mmoles (59.4 mg, 0.20 mmoles) to give the title compound (46.7 mg, 46% yield)
¹H NMR (300 MHz, CDCl₃): δ 8.20 (d, 2H), 7.85 (d, 2H), 7.50 (s, 1H), 6.85-7.05 (m, 5H), 4.45 (t, 2H), 4.00 (s, 3H), 3.88 (s, 3H), 3.85(s, 3H), 2.95 (t, 2H).

### 3-(4-Methyl-benzyl)-2-thioxo-thiazolidine-4-one

Following general procedure B, bis(carboxymethyl) trithiocarbonate (905 mg, 4.0 mmoles), was reacted with 4-methyl-benzyl amine (242 mg, 2.0 mmoles) to give the title compound (250 mg, 53% yield).
¹HNMR (CDCl₃): δ 7.40 (d, 2H), 7.15 (d, 2H), 5.20 (s, 2H), 4.00 (s, 2H), 2.35 (s, 3H).

### 4-{5-[3-(4-Methyl-benzyl)-4-oxo-2-thioxo-thiazolidine-5-ylidenemethyl]-furan-2-yl}-benzoic acid methyl ester

Following general procedure C, 4-(5-formyl-furan-2-yl)-benzoic acid methyl ester (46.0 mg, 0.2 mmoles) was reacted with 3-(4-methyl-benzyl)-2-thioxo-thiazolidine-4-one (47.4 mg, 0.2 mmoles) to give the title compound (46.7 mg, 46% yield).
¹H NMR (300 MHz, CDCl₃): δ 8.20 (d, 2H), 7.85 (d, 2H), 7.50 (s, 1H), 7.40 (d, 1H), 7.15 (d, 1H), 6.90-7.05 (m, 4H), 5.30 (s, 2H), 3.95 (s, 2H), 2.30 (s, 3H).

### Primary biochemical assay for the inhibition of Biot-HIF-1α⁷⁸⁶⁻⁸²⁶/GST-p300^{323/423}

The compounds were evaluated for their ability to inhibit the interaction between Hif-1α and p300 using a fluorescence assay (DELFIA^{™}). The procedure described by Freedman SJ at al., Nature Structural Biology 2003, 10 (7), 504-512 was suitably modified.

The human biotinylated Hif-1α fragment corresponding to C-terminal 786-826 amino acids (Biotinylated Hif-1α ⁷⁸⁶⁻⁸²⁶) was obtained from AnaSpec Inc (San Josè, California, USA) and used without further purifications.

A construct expressing the GST-p30³²³⁻⁴²³ fragment was transformed in the BL21 strain (DE3) of E. coli. Said construct was obtained by cloning in the expression vector pGEX- 4T-1 (Amersham No. 27-45-80-01) the DNA sequence which encodes for the p300 region ranging from amino acids 323 to 423; the DNA sequence was obtained by PCR (Polymerase Chain Reaction). Protein expression was induced with 1 mM isopropyl-1-thio-β-D-galactopyranoside (IPTG). The bacteria were lysed by sonication in the presence of a suitable buffer (50 mM Tris.HCl pH 8.00, 100 mM NaCl, 0.1 mM ZnSO₄, 1 mM DTT, 0.1 mg/ml lysozyme and a tablet of Complete EDTA-free Protease Inhibitor Cocktail Tablets (Roche, catalogue number 1 873 580)) and the GST fusion protein present in the soluble fraction was purified on a Glutathione-Sepharose 4B resin (Amersham Biosciences; no. 27-4574-01). The protein final concentration was determined according to Bradford with the Biorad assay (Bradford M., Anal. Biochem.,72, 248, (1976)) and sample purity was evaluated by SDS-PAGE. The samples were stored at -80°C in 50% glycerol.

The assay was carried out using NUNC Maxisorp 96-well plates as follows.

C96 NUNC Maxisorp plates (from Nunc, product No. 446612) were incubated overnight with streptavidin (Sigma; product No. S 4762) at a final concentration of 1 µg/ml in PBS buffer (Phosphate Buffered Saline 10 mM sodium phosphate, 150 mM sodium chloride pH 7.4). Each well was subsequently washed with 3x300 µl of TBST buffer (50 mM Tris-HCl pH 8.0, 150 mM NaCl, 0.05% (v/v) Tween 20). Each well was then added with 100 µl of a 10 nM solution of biotinylated Hif-1α⁷⁸⁶⁻⁸²⁶ in TBSB (50 mM Tris-HCl pH 8.0, 150 mM NaCl, 5% (w/v) BSA (Sigma, product No. A 2153)) and incubated for 1 h at 25°C. In the last row of each plate, TBSB buffer only was added. Each well was subsequently washed with 3 x 300 µl of TBST buffer. The thus prepared plate represented the assay plate.

Separately, a plate (daughter plate) containing in each well 10 µl of each test compound dissolved in DMSO to a concentration of 10 µM was prepared. This plate was added with 100 µl of a 111 pM solution of GST-p300³²³⁻⁴²³ diluted in the incubation buffer (TBSB added with 0.1% (v/v) Tween 20, 0.5 mM DTT, 10 µM ZnCl₂) and the whole was mixed. 100 µl of the mixture contained in the daughter plate was immediately transferred to the assay plate.

Each daughter plate was prepared with 80 different compounds at a 10 µM concentration, safe for the last two well rows, wherein each well was added with 10 µl of DMSO. These two rows represented the positive (row 11, + Hif-1) and negative (row 12, - Hif-1) controls.

After incubation for 1 h at 25°C, each well was washed with 3 x 300 µl of TBST buffer (50 mM Tris-HCl pH 8.0, 150 mM NaCl, 0.05% (v/v) Tween 20). Each well was then added with 60.8 ng of an europium-labelled anti-GST antibody (DELFIA Eu-N1 labeled; Perkin Elmer; product no. A D 0251) dissolved in 100 µl of TBST buffer containing 10 µM ZnCl₂. After incubation for 1 h at room temperature, each well was washed with 3 x 300 µl of TBST buffer, then 100 µl of signal amplification solution (Enhancement Solution, Perkin Elmer product No. 1244-105) was added.

The plates were then read using a FUSION alpha-FP-HT reader (Perkin Elmer) in fluorescence mode for time resolution.

The activity of the compounds was calculated as follows. The fluorescence mean value of negative controls in row 12 of the plate test was subtracted from the fluorescence value of all the other wells. The resulting fluorescence value for each well was then divided by the fluorescence mean value of the positive controls in row 11 (which represent the maximum signal value, 100%) and expressed as percentage value. The inhibition value was expressed as the difference to 100 of the signal percentage calculated for each well.

Using daughter plates in which the compounds were present at 10 different concentrations ranging from 90 µM to 0.178 µM in each row, a dose-response curve could be calculated from which the IC₅₀ value (concentration of the compound necessary to inhibit the signal by 50%) was obtained. Rows 11 and 12 containing the vehicle only were the controls.

The IC₅₀ values obtained for some compounds of the present invention are reported in Table 1. The data are the mean of two independent experiments.

**Table 1**

| **Structure** | **Compound** | **HIF/p300 (IC₅₀, µm)** |
|---|---|---|
| 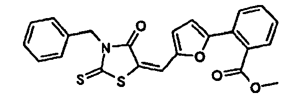 | 1 | 8.6 |
| 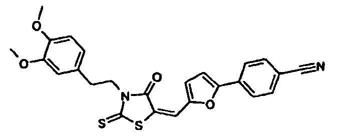 | 18 | 29.9 |
| 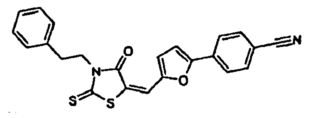 | 19 | 1.4 |
| 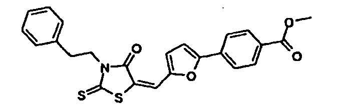 | 20 | 4.2 |
| 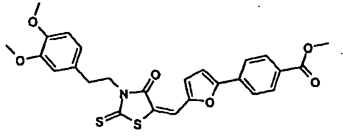 | 21 | 7.4 |
| 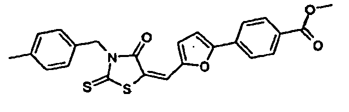 | 9 | 5.9 |

### Inhibition of VEGF production

The compounds having inhibitory activity in the Hif-1α/p300 assay described above were evaluated using a cellular test on genetically modified human hepatocarcinoma Hep3B cells (Hep3B-VEGF*Luciferase*) in order to stably express a vector in which the Open Reading Frame of *firefly Luciferase* is under the control of the rat VEGF gene promoter.

Hif-1 induction by deferoxamine (which causes hypoxia) induces luciferase transcription through activation of the VEGF promoter, which in turn increases luciferase activity, which can be measured with a commercially available kit. The compounds interfering with the Hif-1α/p300 complex inhibit Hif-dependent luciferase activation, resulting in a reduction of luciferase activity. Therefore, this assay allows to evaluate the activity of the compounds towards the VEGF promoter, which is essential to VEGF production and subsequent tumor angiogenesis.

The Hep-3B-VEGF *Luciferase* cell line was obtained according to the following procedure.

Human hepatocarcinoma cells Hep-3B (ATCC reference No. HB-8064) were seeded onto 6-well plates at a concentration of 2.5 x 10⁵ cells/well in 2 ml DMEM/10% FCS and the following day were transfected using Fugene 6 (Roche Biochemicals®). In each well, the transfection mixtures contained 6 µl of the transfection reagent Fugene 6, 1 µg of the pxp2-VEGF-luciferase reporter plasmid (VEGF rat promoter, NCBI GenBank No. of accession U22373, Levy et al., J. Biol. Chem. 270 (22), 13333-13340, 1995), and 10 ng of pcDNA3.1(+)plasmid (INVITROGEN) which makes the cells resistant to neomycin. Transfection was carried out according to the manufacturer's instructions.

A suitable cell population (phenotypically resistant to neomycin) was selected by means of a cloning approach based on the "dilution limit" procedure (Sambrook J., Fritsch E.F. and Maniatis T. (1989) Molecular Cloning, A Laboratory Manual; Cold Spring Harbor Laboratories). The subsequent assays for Luciferase expression/activity (Luciferase assay) and for the quantification of secreted VEGF in the supernatant (ELISA secreted VEGF test) were carried out with the stably transfected selected cells.

The following experimental protocol was used:

Day 1. Hep-3B -VEGF Luciferase cells were seeded onto "blank" 96-wells plates (Greiner) at a density of 1 x 10⁴ cells/well/125 µl of medium, then left to adhere overnight in thermostat (37°C/5% CO₂).

Day 2. 75 µl of "3.2x working solutions" of compound (previously prepared in culture medium so that the DMSO concentration is 1.6% v/v) were added to the cells (partial volume/well = 200 µl, partial concentration of compound = 1.2 x, partial concentration of DMSO = 0.6%). After 1 hour incubation in thermostat, hypoxia was induced chemically by addition of 40 µl/well of a 6x (600 µM) stock solution of deferoxamine (final volume/well = 240 µl, final concentration of compound = 1x, final concentration of DMSO = 0.5%, final concentration of deferoxamine = 1x ≈ 100 µM). The plates were then thermostated for further 18-20 hours.

Day 3. The Luciferase assay and the secreted VEGF ELISA test were carried out as follows.

### Secreted VEGF ELISA test

Quantification of secreted VEGF was performed using the "DuoSet Elisa Development System human VEGF" kit (R&D Systems). 100 µl/well of the supernatant from the "blank" 96-well plates with the cells of the Hep3B/VEGF Luciferase clone were transferred into transparent 96-well plates (Maxisorp) and assayed according to the indications of the kit manufacturer.

### Luciferase assay

Quantification of the expression of the Luciferase reporter gene was performed by means of the Bright Glo Reagent (Promega). After removing the supernatant and washing once with PBS, 40 µl/well of Bright Glo Reagent were added to blank 96-well plates with Hep3B/VEGF-Luciferase cells. The expression levels of the reporter gene were determined by reading the plates with a luminometer.

IC₅₀ values (concentration of the compound that causes 50% inhibition of the luciferase signal or 50% reduction of secreted VEGF) for some compounds of the invention are reported in table 2.

**Table 2**

| | | **IC₅₀ (µM)** | |
|---|---|---|---|
| **Compound** | **Structure** | **Luciferase assay** | **ELISA test (secreted VEGF)** |
| 1 | 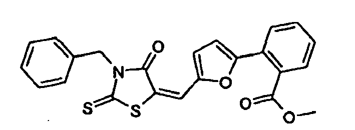 | 6.66 | 0.37 |
| 18 | 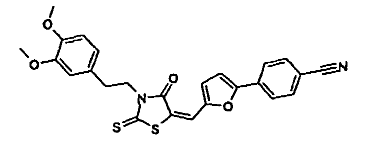 | 3.17 | 0.49 |
| 19 | 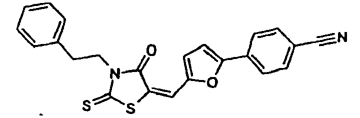 | 7.29 | 0.33 |
| 20 | 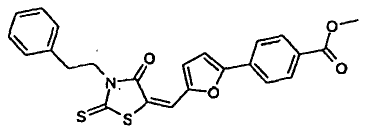 | 10.47 | 0.46 |
| 21 | 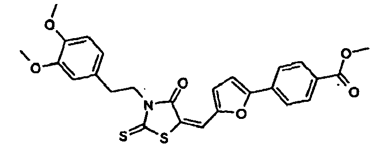 | 4.86 | 0.24 |
| 9 | 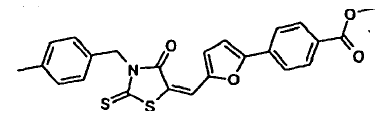 | > 5.0 | 0.18 |

### Bibliography

1) G.I. Semenza, Nature Review Cancer, 3, October 2003, 721-732.
2) Semenza GL. HIF-1 and tumor progression: pathophysiology and therapeutics. Trends Mol.. Med. 2002 8:S62.
3) Semenza GL et al. Dimerization, DNA binding, and transactivation properties of hypoxia-inducible factor 1. J. Biol. Chem. 1996 271:17771.
4) Semenza GL et al. Hypoxia-inducible factor 1 levels vary exponentially over a physiologically relevant range of O2 tension. Am. J. Physiol. 1996 271:C1172.
5) Epstein AC et al. C. elegans EGL-9 and mammalian homologs define a family of dioxygenases that regulate HIF by prolyl hydroxylation. Cell 2001 107:43.
6) Semenza GL et al. FIH-1: a novel protein that interacts with HIF-1 alpha and VHL to mediate repression of HIF-1 transcriptional activity. Genes Dev. 2001 15:2675.
7) Arany Z. et al. An esential role for p300/cbp in the cellular-response to hypoxia. Proc. Nat. Acad. Sci. USA 1996 93; 12969.
8) Damert A. et al. Activator-protein-1 binding potentiates the hypoxia-inducible factor-1-mediated hypoxia induced transcriptional activation of vascular-endothelial growth-factor expression in c6 glioma cells. Biochem J. 1997 327:419.
9) Eck MJ et al. Structural basis for recruitment of CBP/p300 by hypoxia-inducible factor-1 alpha. Proc. Natl. Acad. Sci. USA, 2002 99:5367.
10) Wright PE et al. Structural basis for HIF-1 alpha/CBP recognition in the cellular hypoxic response. PNAS, 2002 99:5271.
11) Freedman, S.J. et al, Nature Structural Biology, 2003, 10(7), 504-12).
12) Zhong, H. et al., Overexpression of hypoxia-inducible factor 1α in common human cancers and their metastases, Cancer Research, 1999, 59, 5830-5.
13) Bos, R. et al., Levels of hypoxia-inducible-factors-1α during breast carcinogeneis, J. Nat. Cancer Inst. 2001, 93, 309-14.
14) Talks, K.I. et al., The expression and distribution of the hypoxia-inducible-factors HIF-1α and HIF-2 in normal human tissues, cancers, and tumor-associated macrophages, Am. J. Pathol., 2000, 157, 411-21).
15) Giatromanolaki, A. et al. Relation of hypoxia inducible factor 1α and 2a in operable non-small cell lung cancer to angiogenic/molecular profile of tumours and survival. Br. J.Cancer 85, 881-890 (2001).
16) Aebersold, D. M. et al. Expression of hypoxia-inducible factor 1α: a novel predictive and prognostic parameter in the radiotherapy of oropharyngeal cancer. Cancer Res. 61, 2911-2916 (2001).
17) Birner, P. et al. Overexpression of hypoxia-inducible factor 1α is a marker for an unfavorable prognosis in early-stage invasive cervical cancer. Cancer Res. 60, 4693-4696 (2000).
18) Koukourakis, M. I. et al. Hypoxia-inducible factor (HIF-1 alpha and HIF-2 alpha), angiogenesis, and chemoradiotherapy outcome of squamous cell head-and-neck cancer. Int. J. Radiat. Oncol. Biol. Phys. 53, 1192-1202 (2002).
19) Birner, P. et al. Expression of hypoxia-inducible factor 1α in epithelial ovarian tumors: its impact on prognosis and on response to chemotherapy. Clin. Cancer Res. 7, 1661-1668 (2001).
20) Birner, P. et al. Expression of hypoxia-inducible factor-1α in oligodendrogliomas: its impact on prognosis and on neoangiogenesis. Cancer 92, 165-171 (2001).
21) Koukourakis, M.I. et al. Hypoxia inducible factor (HIF-1α and HIF-2a) expression in early esophageal cancer and response to photodynamic therapy and radiotherapy. Cancer Res. 61, 1830-1832 (2001).
22) Zundel, W. et al. Loss of PTEN facilitates HIF-1-mediated gene expression. Genes Dev. 14, 391-396 (2000).
23) Hudson, C.C. et al. Regulation of hypoxia-inducible factor 1 alpha expression and function by the mammalian target of rapamycin. Mol.. Cell. Biol. 22, 7004-7014 (2002).
24) Sodhi, A. et al. The Kaposi's sarcoma-associated herpes virus G protein-coupled receptor up-regulates vascular endothelial growth factor expression and secretion through mitogen-activated protein kinase and p38 pathways acting on hypoxia-inducible factor 1α. Cancer Res. 60, 4873-4880 (2000).
25) Mabjeesh, N. J. et al. Geldanamycin induces degradation of hypoxia-inducible factor 1α protein via the proteosome pathway in prostate cancer cells. Cancer Res. 62, 2478-2482 (2002).
26) Welsh, S. J. et al. The thioredoxin redox inhibitors 1-methylpropil 2-imidazolyl disulfide and pleurotin inhibit hypoxia-induced factor 1α and vascular endothelial growth factor formation. Mol.. Cancer Ther. 2, 235-243 (2003).
27) Mabjeesh, N. J. et al. 2ME2 inhibits tumor growth and angiogenesis by disrupting microtubules and dysregulating HIF. Cancer Cell 3, 363-375 (2003).
28) Escuin, D. et al., Epothilone B inhibits HIF-1α downstream of its microtubule stabilizing effects; Proceedings of the 95th Annual Meeting of the American Association for Cancer Research, Abs. 5427.
29) Welsh, S. et al., Antitumor activity and pharmacodynamic properties of PX-478, an inhibitor of hypoxia-inducible factor-1 alpha Mol. Cancer Ther. 2004;3(3):233-244).
30) A.L. Kung et al., Small molecule blockade of transcriptional coactivation of the hypoxia-inducible factor pathway. Cancer Cell, 6, 33-43, 2004.

## Claims

1. Use of a compound selected from
2-[5-(3-benzyl-4-oxo-2-thioxo-thiazolidine-5-ylidenemethyl)-furan-2-yl]benzoic acid methyl ester (**1**);
4-[5-(3-benzyl-4-oxo-2-thioxo-thiazolidine-5-ylidenemethyl)furan-2-yl-] benzonitrile (**5**);
4-{5-[3-(4-methylbenzyl)-4-oxo-2-thioxo-thiazolidine-5-ylidenemethyl]furan-2-yl-}benzonitrile (**6**);
4-{5-[3-(4-methylbenzyl)-4-oxo-2-thioxo-thiazolidine-5-ylidenemethyl]-furan-2-yl}benzoic acid methyl ester (**9**);
4-{5-[3-(3,4-methylenedioxybenzyl)-4-oxo-2-thioxo-thiazolidine-5-ylidenemethyl]-furan-2-yl}benzenesulfonamide (**11**);
4-{5-[3-(3,4-methylenedioxybenzyl)-4-oxo-2-thioxo-thiazolidine-5-ylidenemethyl]-furan-2-yl}benzoic acid methyl ester (**12**);
4-{5-[3-(2-thienyl)methyl-4-oxo-2-thioxo-thiazolidine-5-ylidenemethyl]-furan-2-yl}benzonitrile (**14**);
4-{5-[3-(2-thienyl)methyl-4-oxo-2-thioxo-thiazolidine-5-ylidenemethyl]-furan-2-yl}benzenesulfonamide (**15**);
4-{5-[3-(3,4-dimethoxyphenyl)-4-oxo-2-thioxo-thiazolidine-5-ylidenemethyl]-furan-2-yl}benzoic acid (**17**);
4-{5-[3-[2-(3,4-dimethoxyphenyl)ethyl]-4-oxo-2-thioxo-thiazolidine-5-ylidenemethyl]-furan-2-yl}benzonitrile (**18**);
4-{5-[3-[2-(phenyl)ethyl]-4-oxo-2-thioxo-thiazolidine-5-ylidenemethyl]-furan-2-yl}benzonitrile (**19**);
4-{5-[3-[2-(phenyl)ethyl]-4-oxo-2-thioxo-thiazolidine-5-ylidenemethyl]-furan-2-yl}benzoic acid methyl ester (**20**);
4-{5-[3-[2-(3,4-dimethoxyphenyl)ethyl]-4-oxo-2-thioxo-thiazolidine-5-ylidenemethyl]-furan-2-yl}benzoic acid methyl ester (**21**);
2-{5-[4-[3-[2-(phenyl)ethyl]-4-oxo-2-thioxo-thiazolidine-5-ylidenemethyl]-furan-2-yl]phenoxy}ethanol (**22**);
3-benzyl-5-[[5-(2-methoxyphenyl)furan-2-ylidenemethyl]-4-oxo-2-thioxo-thiazolidine (**23**); and
2-[5-(3-benzyl-4-oxo-2-thioxo-thiazolidine-5-ylidenemethyl)-furan-2-yl]benzoic acid (**24**),
for the preparation of pharmaceutical compositions for the treatment of solid tumors.

2. Use as claimed in claim 1 in which the tumors are selected from lung carcinoma, mammary carcinoma, prostate carcinoma, neuroblastoma, glioblastoma multiforme, melanoma, central nervous system cancer, squamous cell oropharyngeal cancer, cervical cancer, ovary, esophageal, kidney, colon, head-and-neck cancer and oligodendrioglioma.

3. A compound selected from:
4-[5-(3-benzyl-4-oxo-2-thioxo-thiazolidine-5-ylidenemethyl)furan-2-yl-] benzonitrile (**5**);
4-{5-[3-(4-methylbenzyl)-4-oxo-2-thioxo-thiazolidine-5-ylidenemethyl]furan-2-yl-}benzonitrile (**6**);
4-{5-[3-(4-methylbenzyl)-4-oxo-2-thioxo-thiazolidine-5-ylidenemethyl]-furan-2-yl}benzoic acid methyl ester (**9**);
4-{5-[3-(3,4-methylenedioxybenzyl)-4-oxo-2-thioxo-thiazolidine-5-ylidenemethyl]-furan-2-yl}benzenesulfonamide (**11**);
4-{5-[3-(3,4-methylenedioxybenzyl)-4-oxo-2-thioxo-thiazolidine-5-ylidenemethyl]-furan-2-yl}benzoic acid methyl ester (**12**);
4-{5-[3-(2-thienyl)methyl-4-oxo-2-thioxo-thiazolidine-5-ylidenemethyl]-furan-2-yl}benzonitrile (**14**);
4-{5-[3-(2-thienyl)methyl-4-oxo-2-thioxo-thiazolidine-5-ylidenemethyl]-furan-2- yl}benzenesulfonamide (**15**);
4-{5-[3-(3,4-dimethoxyphenyl)-4-oxo-2-thioxo-thiazolidine-5-ylidenemethyl]-furan-2-yl}benzoic acid (**17**);
4-{5-[3-[2-(3,4-dimethoxyphenyl)ethyl]-4-oxo-2-thioxo-thiazolidine-5-ylidenemethyl]-furan-2-yl}benzonitrile (**18**);
4-{5-[3-[2-(phenyl)ethyl]-4-oxo-2-thioxo-thiazolidine-5-ylidenemethyl]-furan-2-yl}benzonitrile (**19**);
4-{5-[3-[2-(phenyl)ethyl]-4-oxo-2-thioxo-thiazolidine-5-ylidenemethyl]-furan-2-yl}benzoic acid methyl ester (**20**);
4-{5-[3-[2-(3,4-dimethoxyphenyl)ethyl]-4-oxo-2-thioxo-thiazolidine-5-ylidenemethyl]-furan-2-yl}benzoic acid methyl ester (**21**);
2-{5-[4-[3-[2-(phenyl)ethyl]-4-oxo-2-thioxo-thiazolidine-5-ylidenemethyl]-furan-2-yl]phenoxy}ethanol (**22**);
3-benzyl-5-[[5-(2-methoxyphenyl)furan-2-ylidenemethyl]-4-oxo-2-thioxo-thiazolidine (**23**); and
2-[5-(3-benzyl-4-oxo-2-thioxo-thiazolidine-5-ylidenemethyl)-furan-2-yl]benzoic acid (**24**)

## Patentansprüche

1. Verwendung einer Verbindung, ausgewählt aus
2-[5-(3-Benzyl-4-oxo-2-thioxo-thiazolidin-5-ylidenmethyl)-furan-2-yl]benzoesäuremethylester (1);
4-[5-(3-Benzyl-4-axo-2-thioxo-thiazolidin-5-ylidenmethyl)furan-2-yl-]benzonitril (5);
4-{5-[3-(4-Methylbenzyl-)-4-oxo-2-thioxo-thiazolidin-5-ylidenmethyl]furan-2-yl-}benzonitril (6);
4-{5-[3-(4-Methylbenzyl)-4-oxo-2-thioxo-thiazolidin-5-ylidenmethyl]-furan-2-yl}benzoesäuremethylester (9);
4-{5-[3-(3,4-Methylendioxybenzyl)4-oxo-2-thioxo-thiazolidin-5-ylidenmethyl]-furan-2-yl}benzolsulfonamid (11);
4-{5-[3-(3,4-Methylendioxybenzyl)-4oxo2-thioxo-thiazolidin-5-ylidenmethyl]-furan-2-yl}benzoesäuremethylester (12);
4-{5-[3-(2-Thienyl)methyl-4-oxo-2-thioxo-thiazolidin-5-ylidenmethyl]-furan-2-yl}benzonitril (14);
4-{5-[3-(2-Thienyl)methyl-4-oxo-2-thioxo-thiazolidin-5-ylidenmethyl]-furan-2-yl}benzolsulfonamid (15);
4-{5-[3-(3,4-Dimethoxyphenyl)-4-oxo-2-thioxo-thiazolidin-5-ylidenmethyl]-furan-2-yl}benzoesäure (17);
4-{5-[3-[2-(3,4-Dimethoxyphenyl)ethyl]-4-oxo-2-thioxo-thiazolidin-5-ylidenmethyl]-furan-2-yl}benzonitril (18);
4-{5-[3-[2-(Phenyl)ethyl]-4-oxo-2-thioxo-thiazolidin-5-ylidenmethyl]-furan-2-yl}benzonitril (19);
4-{5-[3-[2-(Phenyl)ethyl]-4-oxo-2-thioxo-thiazolidin-5-ylidenmethyl]-furan-2-yl}benzoesäuremethylester (20);
4-(5-[3-[2-(3,4-Dimethoxyphenyl)ethyl]-4-oxo-2-thioxo-thiazolidin-5-ylidenmethyl]-furan-2-yl}benzoesäuremethylester (21);
2-{5-[4-[3-[2-(Phenyl)ethyl]-4-oxo-2-thioxo-thiazolidin-5-ylidenmethyl]-furan-2-yl]phenoxy}ethanol (22);
3-Benzyl-5-[[5-(2-methoxyphenyl)furan-2-ylidenmethyl]-4-oxo-2-thioxo-thiazolidin (23) und
2-[5-(3-Benzyl-4-oxo-2-thioxo-thiazolidin-5-ylidenmethyl)-furan-2-yl]benzoesäure (24)
zur Herstellung von pharmazeutischen Zusammensetzungen für die Behandlung von soliden Tumoren.

2. Verwendung, wie sie in Anspruch 1 beansprucht ist, wobei die Tumore ausgewählt sind aus Lungenkarzinom, Brustkarzinom, Prostatakarzinom, Neuroblastom, Glioblastoma multiforme, Melanom, Krebs des Zentralnervensystems, oropharyngealem Platten(epithel)zellenkrebs, Cervixkrebs, Eierstockkrebs, Ösophaguskarzinom, Nierenkrebs, Kolonkrebs, Krebs des Kopfes und des Halses und Oligodendriogliom.

3. Verbindung, ausgewählt aus:
4-[5-(3-Benzyl-4-oxo-2-thioxo-thiazolidin-5-ylidenmethyl)furan-2-yl-]benzonitril (5);
4-{5-[3-(4-Methylbenzyl)-4-oxo-2-thioxo-thiazolidin-5-ylidenmethyl]furan-2-yl-}benzonitril (6);
4-{5-[3-(4-Methylbenzyl)-4-oxo-2-thioxo-thiazolidin-5-ylidenmethyl]-furan-2-yl}benzoesäuremethylester (9);
4-{5-[3-(3,4-Methylendioxybenzyl)-4-oxo-2-thioxo-thiazolidin-5-ylidenmethyl]-furan-2-yl}benzolsulfonamid (11);
4-{5-[3-(3,4-Methylendioxybenzyl)-4-oxo-2-thioxo-thiazolidin-5-ylidenmethyl]-furan-2-yl}benzoesäuremethylester (12);
4-{5-[3-(2-Thienyl)methyl-4-oxo-2-thioxo-thiazolidin-5-ylidenmethyl]-furan-2-yl}benzonitril (14);
4-{5-[3-(2-Thienyl)methyl-4-oxo-2-thioxo-thiazolidin-5-ylidenmethyl]-furan-2-yl}benzolsulfonamid (15);
4-{5-[3-(3,4-Dimethoxyphenyl)"4-oxo"2-thioxo-thiazolidin-5-ylidenmethyl]-furan-2-yl}benzoesäure (17);
4-{5-[3-[2-(3,4-Dimethoxyphenyl)ethyl]-4-oxo-2-thioxo-thiazolidin-5-ylidenmethyl]-furan-2-yl}benzonitril (18);
4-{5-[3-[2-(Phenyl)ethyl]-4-oxo-2-thioxo-thiazolidin-5-ylidenmethyl]-furan-2-yl}benzonitril (19);
4-{5-[3-[2-(Phenyl)ethyl]-4-oxo-2-thioxo-thiazolidin-5-ylidenmethyl]-furan-2-yl}benzoesäuremethylester (20);
4-{5-[3-[2-(3,4-Dimethoxyphenyl)ethyl]-4-oxo-2-thioxo-thiazolidin-5-ylidenmethyl]-furan-2-yl}benzoesäuremethylester (21);
2-{5-[4-[3-[2-(Phenyl)ethyl]-4-oxo-2-thioxo-thiazolidin-5-ylidenmethyl]-furan-2-yl]phenoxy}ethanol (22);
3-Benzyl-5-[[5-(2-methoxyphenyl)furan-2-ylidenmethyl]-4-oxo-2-thioxo-thiazolidin (23) und
2-[5-(3-Benzyl-4-oxo-2-thioxo-thiazolidin-5-ylidenmethyl)-furan-2-yl]benzoesäure (24).

## Revendications

1. Utilisation d'un composé choisi parmi :
l'ester méthylique de l'acide 2-[5-(3-benzyl-4-oxo-2-thioxo-thiazolidine-5-ylidèneméthyl)-furan-2-yl]benzoïque (1) ;
le 4-[5-(3-benzyl-4-oxo-2-thioxo-thiazolidine-5-ylidèneméthyl)furan-2-yl-]benzonitrile (5) ;
le 4-{5-[3-(4-méthylbenzyl)-4-oxo-2-thioxo-thiazolidine-5-ylidèneméthyl]-furan-2-yl-}benzonitrile (6) ;
l'ester méthylique de l'acide 4-{5-[3-(4-méthylbenzyl)-4-oxo-2-thioxo-thiazolidine-5-ylidèneméthyl]-furan-2-yl} benzoïque (9) ;
le 4-{5-[3-(3,4-méthylènedioxybenzyl)-4-oxo-2-thioxo-thiazolidine-5-ylidèneméthyl]-furan-2-yl}benzènesulfonamide (11) ;
l'ester méthylique de l'acide 4-{5-[3-(3,4-méthylènedioxybenzyl)-4-oxo-2-thioxo-thiazolidine-5-ylidèneméthyl]-furan-2-yl} benzoïque (12) ;
le 4-{5-[3-(2-thiényl)méthyl-4-oxo-2-thioxo-thiazolidine-5-ylidèneméthyl]-furan-2-yl}benzonitrile (14) ;
le 4-{5-[3-(2-thiényl)méthyl-4-oxo-2-thioxo-thiazolidine-5-ylidéneméthyl]-furan-2-yl}benzènesulfonamide (15) ;
l'acide 4- {5-[3-(3,4-diméthoxyphényl)-4-oxo-2-thioxo-thiazolidine-5-ylidèneméthyl]-furan-2-yl}benzoïque (17) ;
le 4-{5-[3-[2-(3,4-diméthoxyphényl)éthyl]-4-oxo-2-thioxo-thiazolidine-5-ylidèneméthyl]-furan-2-yl}benzonitrile (18) ;
le 4-{5-[3-[2-(phényl)éthyl]-4-oxo-2-thioxo-thiazolidine-5-ylidèneméthyl]-furan-2-yl}benzonitrile (19) ;
l'ester méthylique de l'acide 4-{5-[3-[2-(phényl)éthyl]-4-oxo-2-thioxo-thiazolidine-5-ylidèneméthyl]-furan-2-yl} benzoïque (20) ;
l'ester méthylique de l'acide 4-{5-[3-[2-(3,4-diméthoxyphényl)éthyl]-4-oxo-2-thioxo-thiazolidine-5-ylidèneméthyl]-furan-2-yl} benzoïque (21) ;
le 2-{5-[4-[3-[2-(phényl)éthyl]-4-oxo-2-thioxo-thiazolidine-5-ylidèneméthyl]-furan-2-yl]phénoxy}éthanol (22) ;
la 3-benzyl-5-[5-(2-méthoxyphényl)furan-2-ylidèneméthyl]-4-oxo-2-thioxo-thiazolidine (23) ; et
l'acide 2-[5-(3-benzyl-4-oxo-2-thioxo-thiazolidine-5-ylidèneméthyl)-furan-2-yl]benzoïque (24),
pour la préparation de compositions pharmaceutiques destinées au traitement de tumeurs solides.

2. Utilisation selon la revendication 1, dans laquelle les tumeurs sont choisies parmi un carcinome du poumon, un carcinome du sein, un carcinome de la prostate, un neuroblastome, un glioblastome multiforme, un mélanome, le cancer du système nerveux central, le cancer oropharyngé des cellules squameuses, le cancer du col de l'utérus, des ovaires, de l'oesophage, des reins, du côlon, de la tête et du cou et un oligodendriogliome.

3. Composé choisi parmi :
le 4-[5-(3-benzyl-4-oxo-2.thioxo-thiazolidine-5-ylidèneméthyl)furan-2-yl-]benzonitrile (5) ;
le 4-{5-[3-(4-méthylbenzyl)-4-oxo-2-thioxo-thiazolidine-5-ylidèneinéthyl]-furan-2-yl-}benzonitrile (6) ;
l'ester méthylique de l'acide 4-{5-[3-(4-méthylbenzyl)-4-oxo-2-thioxo-thiazolidine-5-ylidèneméthyl]-furan-2-yl}benzoïque (9) ;
le 4-{5-[3(3,4-méthylènedioxybenzyl)-4-oxo-2-thioxo-thiazolidine-5-ylidèneméthyl]-furan-2-yl}benzènesulfonamide (11) ;
l'ester méthylique de l'acide 4-{5-[3-(3,4-méthylènedioxybenzyl)-4-oxo-2-thioxo-thiazolidine-5-ylidèneméthyl]-furan-2-yl}benzoïque (12) ;
le 4-{5-[3-(2-thiényl)méthyl-4-oxo-2-thioxo-thiazolidine-5-ylidèneméthyl]-furan-2-yl}benzonitrile (14);
le 4-{5-[3-(2-thiényl)méthyl-4-oxo-2-thioxo-thiazolidine-5-ylidèneméthyl]-furan-2-yl}benzènesulfonamide (15) ;
l'acide 4-{5-[3-(3,4-diméthoxyphényl)-4-oxo-2-thioxo-thiazolidine-5-ylideneméthyl]-furan-2-yl} benzoïque (17) ;
le 4-{5-[3-[2-(3,4-diméthoxyphényl)éthyl]-4-oxo-2-thioxo-thiazolidine-5-ylidèneméthyl]-furan-2-yl}benzonitrile (18) ;
le 4-{5-[3-[2-(phényl)éthyl]-4-oxo-2-thioxo-thiazolidine-5-ylidèneméthyl]-furan-2-yl}benzonitrile (19) ;
l'ester méthylique de l'acide 4-{5-[3-[2-(phényl)éthyl]-4-oxo-2-thioxo-thiazolidine-5-ylidèneinéthyl]-furan-2-yl}benzoïque (20) ;
l'ester méthylique de l'acide 4-{5-[3-[2-(3,4-diméthoxyphényl)éthyl]-4-oxo-2-thioxo-thiazolidine-5-ylidèneméthyl]-furan-2-yl}benzoïque (21) ;
le 2-{5-[4-[3-[2-(phényl)éthyl]-4-oxo-2-thioxo-thiazolidine-5-ylidèneméthyl]-furan-2-yl]phénoxy} éthanol (22) ;
la 3-benzyl-5-[5-(2-méthoxyphényl)iuran-2-ylidèneméthyl]-4-oxo-2-thioxo-thiazolidine (23) ; et
l'acide 2-[5-(3-benzyl-4-oxo-2-thioxo-thiazolidine-5-ylidèneméthyl)-furan-2-yl]benzoïque (24).
